# EUROPÄISCHE PATENTANMELDUNG

(11) **EP 3 434 786 A1**
(43) Veröffentlichungstag der Anmeldung: **30.01.2019**
(21) Anmeldenummer: 18185574.3
(22) Anmeldetag: 25.07.2018
(51) Int. Cl.: C12Q 1/68

(54) **VERFAHREN ZUR HERSTELLUNG UND FÄLSCHUNGSSICHEREN AUTHENTIFIZIERUNG EINES MEDIZINPRODUKTS DURCH VERWENDUNG EINER NUKLEINSÄURE MIT BEKANNTER TEILSEQUENZ**

(30) Priorität: 28.07.2017 DE 102017007181
(71) Anmelder: FRAUNHOFER-GESELLSCHAFT zur Förderung der angewandten Forschung e.V., 80686 München (DE)
(72) Erfinder: Storsberg, Joachim, 55286 Wörrstadt (DE); Volkert, Marina, 13503 Berlin (DE)
(74) Vertreter: Maiwald Patent- und Rechtsanwaltsgesellschaft mbH

(57) **Zusammenfassung**

Die Erfindung betrifft ein Verfahren zur Herstellung und fälschungssicheren Authentifizierung von Medizinprodukten durch Verwendung einer Nukleinsäure mit bekannter Teilsequenz, wobei das Verfahren die folgenden Schritte aufweist:
(i) Herstellen eines Medizinprodukts (MED) mit einer permanenten Markierung (PM), wobei das Medizinprodukt (MED) ein Polymer (P) mit der permanenten Markierung (PM) umfasst und die permanente Markierung (PM) mindestens eine Nukleinsäure (NUK) mit einer bekannten Teilsequenz (TSEQ) enthält;
(ii) Isolieren der permanenten Markierung (PM); und
(iii) Authentifizierung des Medizinprodukts (MED) durch Nachweis der mindestens einen bekannten Teilsequenz (TSEQ).

SEQUENZPROTOKOLL

<110> Fraunhofer-Gesellschaft zur Förderung der angewandten
      Forschung e.V.

<120> Verfahren zur Herstellung und fälschungssicheren
      Authentifizierung eines Medizinprodukts durch Verwendung einer
      Nukleinsäure mit bekannter Teilsequenz

<130> F09346EP

<160> 4

<170> PatentIn version 3.5

<210> 1
<211> 20
<212> DNA
<213> Solanum lycopersicum

<400> 1
tactggtggt tttgaagctg                                                    20


<210> 2
<211> 24
<212> DNA
<213> Solanum lycopersicum

<400> 2
aacttccttc acgatttcat cata                                               24


<210> 3
<211> 22
<212> DNA
<213> Solanum lycopersicum

<400> 3
agaccacgag aacgatattt gc                                                 22


<210> 4
<211> 24
<212> DNA
<213> Solanum lycopersicum

<400> 4
ttcttgcctt ttcatatcca gaca                                               24

## Beschreibung

Die Erfindung betrifft ein Verfahren zur Herstellung und fälschungssicheren Authentifizierung eines Medizinprodukts durch Verwendung einer permanenten Markierung umfassend mindestens eine Nukleinsäure mit einer bekannten Teilsequenz.

Die fälschungssichere Kennzeichnung von Wertgegenständen und Sicherheitsdokumenten durch das äußerliche Auftragen einer Markierungslösung auf Basis von Nukleinsäuren ist bekannt und beispielsweise in der WO 03/038000 A1 beschrieben.

Medizinprodukte stellen typischerweise für den Endverbraucher ein gesundheitliches Risiko und für den Hersteller aufgrund potentieller Schadensersatz oder Regressforderungen ein erhebliches finanzielles Risiko dar. Es besteht ein großes Interesse daran Medizinprodukte so zu gestalten, dass sie mit fälschungssicheren Sicherheitsmerkmalen versehen sind, anhand derer die Echtheit verifiziert werden kann.

Es treten im Zusammenhang mit der fälschungssicheren Kennzeichnung von Medizinprodukten eine Reihe von Schwierigkeiten auf, die bei der fälschungssicheren Kennzeichnung von Wertgegenständen und Sicherheitsdokumenten nicht berücksichtigt werden müssen.
Die Sicherheitsmerkmale sollten möglichst universell einsetzbar sein und eine hohe Fälschungssicherheit aufweisen. Die Sicherheitsmerkmale sollten weder unkontrolliert aus dem Medizinprodukt austreten, noch dessen Eigenschaften negativ beeinflussen.

Eine besondere Herausforderung ist die fälschungssichere Kennzeichnung von Materialien, welche erst durch Dritte zu einem Endprodukt weiterverarbeitet werden.

Diese und weitere Aufgaben werden durch die vorliegende Erfindung gelöst. Die Erfindung ist in den unabhängigen Ansprüchen beschrieben, vorteilhafte Ausgestaltungen sind in den abhängigen Ansprüchen angegeben.

### Zusammenfassung

Beschrieben wird ein Verfahren zur Authentifizierung eines Medizinprodukts (MED), umfassend die folgenden Schritte:
(i) Herstellen eines Medizinprodukts (MED) mit einer permanenten Markierung (PM), wobei das Medizinprodukt (MED) ein Polymer (P) mit der permanenten Markierung (PM) umfasst und die permanente Markierung (PM) mindestens eine Nukleinsäure (NUK) mit einer bekannten Teilsequenz (TSEQ) enthält;
(ii) Isolieren der permanenten Markierung (PM); und
(iii) Authentifizierung des Medizinprodukts (MED) durch Nachweis der mindestens einen bekannten Teilsequenz (TSEQ).

In einer Ausführungsform umfasst der Nachweis die Amplifikation der mindestens einen bekannten Teilsequenz (TSEQ) der mindestens einen Nukleinsäure (NUK).

In einer Ausführungsform umfasst der Nachweis die Sequenzierung der mindestens einen bekannten Teilsequenz (TSEQ) der mindestens einen Nukleinsäure (NUK).

In einer Ausführungsform umfasst der Nachweis die Hybridisierung der mindestens einen bekannten Teilsequenz (TSEQ) der mindestens einen Nukleinsäure (NUK) mit einer Nachweisnukleinsäure.

In einer Ausführungsform umfasst die Authentifizierung des Medizinprodukts (MED) durch Nachweis der mindestens einen bekannten Teilsequenz (TSEQ) die folgende Schritte:
(i) Bestimmen der Konzentration (EK) der permanenten Markierung (PM) in dem Polymer (P) des zu authentifizierenden Medizinprodukts (MED);
(ii) Vergleichen der Konzentration (EK) der permanenten Markierung mit der Konzentration (AK) der permanenten Markierung (PM) des zur Herstellung des Medizinprodukts (MED) verwendeten Polymers (P).

Beschrieben ist außerdem ein Verfahren zur Herstellung eines Medizinprodukts (MED) mit einer permanenten Markierung (PM) umfassend die folgenden Schritte:
(i) Bereitstellen eines Polymers (P) mit einer permanenten Markierung (PM), wobei die permanente Markierung (PM) mindestens eine Nukleinsäure (NUK) mit mindestens einer bekannten Teilsequenz (TSEQ) enthält; und
(ii) Verarbeiten des Polymers (P) zu einem Medizinprodukt (MED).

In einer Ausführungsform umfasst das Verfahren zur Herstellung des Medizinprodukts (MED) die folgenden Schritte:
(i) Bereitstellen von mindestens einem Monomer (M);
(ii) Bereitstellen einer permanenten Markierung (PM) enthaltend mindestens einen Nukleinsäure (NUK) mit einer bekannten Teilsequenz (TSEQ);
(iii) Verteilen der permanenten Markierung (PM) in dem mindestens einem Monomer (M);
(iv) Polymerisieren des mindestens einen Monomers (M), so dass ein Polymer (P) umfassend die permanente Markierung (PM) erhalten wird; und
(v) Verarbeiten des Polymers (P) zu einem Medizinprodukt (MED).

In einer Ausführungsform umfasst das Verfahren zur Herstellung des Medizinprodukts (MED) die folgenden Schritte:
(i) Bereitstellen eines Polymers (P);
(ii) Bereitstellen einer permanenten Markierung (PM);
(iii) Verteilen der permanenten Markierung (PM) in dem Polymer (P); und
(iv) Verarbeiten des Polymers (P) zu einem Medizinprodukt (MED).

In einer Ausführungsform umfasst das Verfahren zur Herstellung des Medizinprodukts (MED) die folgenden Schritte:
(i) Bereitstellen eines Polymers (P);
(ii) Bereitstellen eines Lösemittels (LM);
(iii) Lösen des Polymers (P) in dem Lösemittel (LM);
(iv) Bereitstellen einer permanenten Markierung (PM);
(v) Verteilen der permanenten Markierung (PM) in dem gelösten Polymer (P); und
(vii) Verarbeiten des Polymers (P) zu einem Medizinprodukt (MED).

In den beschriebenen Verfahren kann das in dem Medizinprodukt enthaltene Polymer (P) vernetzt sein.

In den beschriebenen Verfahren kann die Nukleinsäure (NUK) mit einer bekannten Teilsequenz (TSEQ) eine Desoxyribunukleinsäure (DNA) sein.

In den beschriebenen Verfahren kann das Medizinprodukt (MED) ein Medizinprodukt der Klasse III ist.

In den beschriebenen Verfahren kann die permanente Markierung (PM) neben der Nukleinsäure (NUK) mit einer bekannten Teilsequenz (TSEQ) mindestens eine weitere Nukleinsäure enthalten, welche die Teilsequenz (TSEQ) nicht aufweist.

Beschrieben wird ein Medizinprodukt (MED) mit einer permanenten Markierung (PM), umfassend ein Polymer (P) mit einer permanenten Markierung (PM), wobei die permanente Markierung (PM) mindestens eine Nukleinsäure (NUK) mit einer bekannten Teilsequenz (TSEQ) enthält.

Beschrieben wird die Verwendung einer permanenten Markierung (PM), umfassend mindestens eine Nukleinsäure (NUK) mit einer bekannten Teilsequenz (TSEQ) zur Authentifizierung eines Medizinprodukts (MED).

### Detaillierte Beschreibung

Beschrieben ist ein Verfahren zur Authentifizierung eines Medizinprodukts (MED), umfassend die folgenden Schritte:
(i) Herstellen eines Medizinprodukts (MED) mit einer permanenten Markierung (PM), wobei das Medizinprodukt (MED) ein Polymer (P) mit der permanenten Markierung (PM) umfasst, und die permanenten Markierung (PM) mindestens eine Nukleinsäure (NUK) mit einer bekannten Teilsequenz (TSEQ) enthält;
(ii) Isolieren der permanenten Markierung (PM); und
(iii) Authentifizierung des Medizinprodukts (MED) durch Nachweis der mindestens einen bekannten Teilsequenz (TSEQ) in der isolierten mindestens einen Nukleinsäure (NUK).

Das Medizinprodukt ist authentisch wenn die mindestens eine bekannte Teilsequenz (TSEQ) in der mindestens einen Nukleinsäure (NUK) nachgewiesen werden kann, welche in der isolierten permanenten Markierung (PM) erhalten ist.

Der Nachweis kann über ein Verfahren erfolgen bei dem die in der permanenten Markierung (PM) enthaltene mindestens eine bekannte Teilsequenz (TSEQ) zunächst amplifiziert wird.

In dieser Ausführungsform umfasst das Verfahren zur Authentifizierung eines Medizinprodukts (MED) die folgenden Schritte:
(i) Herstellen eines Medizinprodukts (MED) mit einer permanenten Markierung (PM), wobei das Medizinprodukt (MED) ein Polymer (P) mit der permanenten Markierung (PM) umfasst und die permanente Markierung (PM) mindestens eine Nukleinsäure (NUK) mit einer bekannten Teilsequenz (TSEQ) enthält;
(ii) Isolieren der permanenten Markierung (PM);
(iii) Authentifizierung des Medizinprodukts (MED) durch Nachweis der mindestens einen bekannten Teilsequenz (TSEQ) in der mindestens einen Nukleinsäure (NUK), wobei der Nachweis die Amplifikation der mindestens einen bekannten Teilsequenz (TSEQ) der mindestens einen Nukleinsäure (NUK) umfasst.

Das Medizinprodukt ist authentisch wenn die mindestens eine bekannte Teilsequenz (TSEQ) in der mindestens einen Nukleinsäure (NUK) nachgewiesen werden kann, welche in der isolierten permanenten Markierung (PM) enthalten ist und amplifiziert wurde.

Der Nachweis kann aber auch direkt, also ohne Amplifikation der bekannten Teilsequenz (TSEQ), erfolgen.

Es ist beispielsweise möglich die mindestens eine bekannte Teilsequenz (TSEQ) durch Sequenzierung nachzuweisen. Dazu wird die Sequenz der in der isolierten permanenten Markierung (PM) enthaltenen mindestens einen bekannten Teilsequenz (TSEQ) der mindestens einen Nukleinsäure (NUK) ermittelt.
Ein Nachweis durch Sequenzierung muss aber nicht direkt erfolgen, sondern kann neben der Sequenzierung auch die Amplifikation der bekannten Teilsequenz (TSEQ) umfassen.

Demzufolge umfasst das Verfahren zur Authentifizierung eines Medizinprodukts (MED) in einer Ausführungsform die folgenden Schritte:
(i) Herstellen eines Medizinprodukts (MED) mit einer permanenten Markierung (PM), wobei das Medizinprodukt (MED) ein Polymer (P) mit der permanenten Markierung (PM) umfasst und die permanente Markierung (PM) mindestens eine Nukleinsäure (NUK) mit einer bekannten Teilsequenz (TSEQ) enthält;
(ii) Isolieren der permanenten Markierung (PM);
(iii) Authentifizierung des Medizinprodukts (MED) durch Nachweis der mindestens einen bekannten Teilsequenz (TSEQ) in der mindestens einen Nukleinsäure (NUK), wobei der Nachweis die Sequenzierung der mindestens einen bekannten Teilsequenz (TSEQ) der mindestens einen Nukleinsäure (NUK) umfasst.

Es ist weiterhin möglich durch Hybridisierung mit einer Nachweisnukleinsäure die mindestens eine bekannte Teilsequenz (TSEQ) nachzuweisen. Dazu wird die in der isolierten permanenten Markierung (PM) enthaltenen mindestens eine bekannte Teilsequenz (TSEQ) der mindestens einen Nukleinsäure (NUK) mit einer Nachweisnukleinsäure, die zumindest partiell zu der bekannten Teilsequenz (TSEQ) der Nukleinsäure (NUK) komplementär ist, hybridisiert. Ein Nachweis durch Hybridisierung mit einer Nachweisnukleinsäure kann direkt erfolgen, kann aber neben der Hybridisierung mit einer Nachweisnukleinsäure auch die Amplifikation der bekannten Teilsequenz (TSEQ) umfassen.

Demzufolge umfasst das Verfahren zur Authentifizierung eines Medizinprodukts (MED) in einer Ausführungsform die folgenden Schritte:
(i) Herstellen eines Medizinprodukts (MED) mit einer permanenten Markierung (PM), wobei das Medizinprodukt (MED) ein Polymer (P) mit der permanenten Markierung (PM) umfasst und die permanente Markierung (PM) mindestens eine Nukleinsäure (NUK) mit einer bekannten Teilsequenz (TSEQ) enthält;
(ii) Isolieren der permanenten Markierung (PM);
(iii) Authentifizierung des Medizinprodukts (MED) durch Nachweis der mindestens einen bekannten Teilsequenz (TSEQ) in der mindestens einen Nukleinsäure (NUK), wobei der Nachweis die Hybridisierung der mindestens einen bekannten Teilsequenz (TSEQ) der mindestens einen Nukleinsäure (NUK) mit einer Nachweisnukleinsäure umfasst.

Der Begriff "Medizinprodukt (MED)" bezeichnet einen Gegenstand oder einen Stoff, der zu medizinisch therapeutischen oder diagnostischen Zwecken für Menschen verwendet wird, wobei die bestimmungsgemäße Hauptwirkung im Unterschied zu Arzneimitteln primär nicht pharmakologisch, metabolisch oder immunologisch, sondern meist physikalisch oder physikochemisch erfolgt und ist wie folgt definiert:
Medizinprodukte sind alle einzeln oder miteinander verbunden verwendeten Instrumente, Apparate, Vorrichtungen, Stoffe oder anderen Gegenstände, einschließlich der vom Hersteller speziell zur Anwendung für diagnostische und/oder therapeutische Zwecke bestimmten, die vom Hersteller zur Anwendung am Menschen für die Erkennung, Verhütung, Überwachung, Behandlung oder Linderung von Krankheiten; die Erkennung, Überwachung, Behandlung, Linderung oder Kompensierung von Verletzungen oder Behinderungen; die Untersuchung, Ersatz oder Veränderung des anatomischen Aufbaus oder eines physiologischen Vorgangs; oder die Empfängnisregelung bestimmt sind und deren bestimmungsgemäße Hauptwirkung im oder am menschlichen Körper weder durch pharmakologische oder immunologische Mittel noch metabolisch erreicht wird, deren Wirkungsweise aber durch solche Mittel unterstützt werden kann.

Das Medizinprodukt (MED) kann ausgewählt sein aus der Gruppe bestehend aus, Klasse I, i.e. ärztliche Instrumente, Gehhilfen, Rollstühle, Pflegebetten, Stützstrümpfe, Verbandmittel, wiederverwendbare chirurgische Instrumente, OP-Textilien; Klasse IIa, i.e. Dentalmaterialien, Desinfektionsmittel, diagnostische Ultraschallgeräte, Einmalspritzen, Hörgeräte, Kontaktlinsen, Picture Archiving and Communication Systems (PACS), Trachealtuben, Zahnkronen; Klasse IIb, i.e. Anästhesiegeräte, Beatmungsgeräte, Bestrahlungsgeräte, Blutbeutel, Defibrillatoren, Dialysegeräte, Kondome, Kontaktlinsenreiniger, Dentalimplantate, Reinigungsdesinfektionsautomaten; und Klasse III, i.e. Herzkatheter, künstliche Hüft-, Knie-, oder Schultergelenke, Stents, resorbierbares chirurgisches Nahtmaterial, Intrauterinpessar, Brustimplantat, Herzschrittmacher, gemäß Anhang IX der Richtlinie 93/42/EWG.

Insbesondere kann das Medizinprodukt (MED) ausgewählt sein aus der Gruppe bestehend aus Herzkatheter, künstliche Hüft-, Knie-, oder Schultergelenke, Stents, resorbierbares chirurgisches Nahtmaterial, Intrauterinpessar, Brustimplantat, und Herzschrittmacher.

In einer bevorzugten Ausführungsform ist das Medizinprodukt (MED) ein Medizinprodukt der Klasse III.

In einer besonders bevorzugten Ausführungsform ist das Medizinprodukt (MED) ein Brustimplantat.

Das Verfahren zur Herstellung des Medizinprodukts (MED) ist von dem Medizinprodukt selbst abhängig. Die entsprechenden Verfahren sind dem Fachmann bekannt und werden nach Bedarf ausgesucht.

Verfahren zur Herstellung eines Medizinprodukts (MED) mit einer permanenten Markierung (PM) umfassend die folgenden Verfahrensschritte:
(i) Bereitstellen eines Polymers (P) mit einer permanenten Markierung (PM), wobei die permanente Markierung (PM) mindestens eine Nukleinsäure (NUK) mit einer bekannten Teilsequenz (TSEQ) enthält;
(ii) Verarbeiten des Polymers (P) zu einem Medizinprodukt (MED).

Der Begriff "permanente Markierung" bezieht sich auf eine Markierung, welche eingesetzt werden kann um ein qualitatives und/oder quantitatives Authentifizierungsverfahren durchzuführen, wobei die Markierung vorzugsweise dauerhaft mit dem zu authentifizierenden Medizinprodukt verbunden ist.

In anderen Worten kann die permanente Markierung nicht ohne weitere Hilfsmittel aus dem zu authentifizierenden Medizinprodukt entfernt werden. Insbesondere wird bei einem am Menschen angewandten Medizinprodukt die permanente Markierung im Wesentlichen nicht an den Menschen abgegeben. Insbesondere wird bei einem am Menschen angewandten Medizinprodukt die permanente Markierung nicht in pharmakologisch und/oder toxikologisch relevanten Mengen an den Menschen abgegeben.

Der Begriff "permanent" bezieht sich zumindest auf das Medizinprodukt (MED). In einer Ausführungsform bezieht sich der Begriff "permanent" neben dem Medizinprodukt (MED) auch auf das Polymer (P). In anderen Worten ist die permanente Markierung (PM) zumindest mit dem Medizinprodukt (MED) permanent verbunden. In einer Ausführungsform ist die permanente Markierung (PM) außerdem mit dem Polymer (P) permanent verbunden.

Es kann auf unterschiedlichen Wegen erreicht werden, dass die im Polymer enthaltene Markierung im angewandten Medizinprodukt nicht an den Menschen abgegeben wird. Wenn das zur Herstellung des Medizinprodukts (MED) verwendete Polymer (P) umfassend die permanente Markierung (PM) bei der Anwendung am Menschen in direktem Kontakt zum Menschen steht, so führt die Beschaffenheit des Polymer selbst dazu, dass die Markierung im Wesentlichen nicht an den Menschen abgegeben wird, insbesondere nicht in pharmakologisch und/oder toxikologisch relevanten Mengen.

Sollte das Polymer (P) umfassend die permanente Markierung (PM), das zur Herstellung des Medizinprodukts (MED) verwendet wird, bei der Anwendung am Menschen nicht in direktem Kontakt zum Menschen stehen, so kann auch in diesem Fall die Beschaffenheit des Polymer dafür sorgen, dass die permanente Markierung (PM) im Wesentlichen nicht an den Menschen abgegeben wird, insbesondere nicht in pharmakologisch und/oder toxikologisch relevanten Mengen. In einem solchen Fall können aber auch andere Bestandteile des Medizinprodukts (MED) mit Barriereeigenschaften gegenüber der permanenten Markierung (PM) dafür sorgen, dass bei einem am Menschen angewendeten Medizinprodukt die permanente Markierung (PM) im Wesentlichen nicht an den Menschen abgegeben wird, insbesondere nicht in pharmakologisch und/oder toxikologisch relevanten Mengen.

Geeignete Hilfsmittel um die permanente Markierung (PM) aus dem zu authentifizierenden Medizinprodukt (MED) zu entfernen sind beispielsweise Lösemittel, Reagenzien die einen Abbau des Medizinprodukts (MED) bewirken oder eine mechanische Entnahme der permanenten Markierung aus dem Medizinprodukt (MED) durch eine gezielte äußerliche Einwirkung.

Die bekannten Verfahren, i.e. Verfahren zur Herstellung eines Medizinprodukts (MED) mit einer permanenten Markierung (PM), müssen gegebenenfalls leicht modifiziert werden, um die permanente Markierung (PM) in das verwendete Polymer (P) einzuarbeiten.

Typischerweise umfasst ein so modifiziertes Verfahren zur Herstellung eines Medizinprodukts (MED) mit einer permanenten Markierung (PM) die folgenden Schritte:
(i) Bereitstellen von mindestens einem Monomer (M);
(ii) Bereitstellen einer permanenten Markierung (PM) enthaltend mindestens eine Nukleinsäure (NUK) mit einer bekannten Teilsequenz (TSEQ);
(iii) Verteilen der permanenten Markierung (PM) in dem mindestens einem Monomer (M);
(iv) Polymerisieren des mindestens einem Monomer (M), so dass ein Polymer (P) umfassend die permanente Markierung (PM) erhalten wird;
(v) Verarbeiten des Polymers (P) zu einem Medizinprodukt (MED).

Die permanente Markierung (PM) kann dabei vor und/oder während der Polymerisation in dem mindestens einem Monomer (M) verteilt werden.

Alternativ hierzu kann das mindestens eine Monomer (M) zunächst zu einem Polymer (P) umgesetzt und die permanente Markierung (PM) erst im Anschluss an die Polymerisation in das Polymer eingearbeitet werden.

Typischerweise umfasst ein solches Verfahren zur Herstellung eines Medizinprodukts (MED) mit einer permanenten Markierung (PM) die folgenden Schritte:
(i) Bereitstellen eines Polymers (P);
(ii) Bereitstellen einer permanenten Markierung (PM);
(iii) Verteilen der permanenten Markierung (PM) in dem Polymer (P);
(iv) Verarbeiten des Polymers (P) zu einem Medizinprodukt (MED).

In diesem Zusammenhang ist es beispielsweise möglich das Polymer (P) in einem geeigneten Lösemittel zu lösen, die permanente Markierung (PM) in dem gelösten Polymer (P) zu verteilen und das Polymer (P) anschließend aus dem Lösemittel abzuscheiden.

Typischerweise umfasst ein so modifiziertes Verfahren zur Herstellung eines Medizinprodukts (MED) mit einer permanenten Markierung (PM) die folgenden Schritte:
(i) Bereitstellen eines Polymers (P);
(ii) Bereitstellen eines Lösemittels (LM);
(iii) Lösen des Polymers (P) in dem Lösemittel (LM);
(iv) Bereitstellen einer permanenten Markierung (PM);
(v) Verteilen der permanenten Markierung (PM) in dem gelösten Polymer (P);
(vi) Verarbeiten des Polymers (P) zu einem Medizinprodukt (MED).

Es kann vorgesehen sei das Polymer (P) nach dem Verteilen der permanenten Markierung (PM) in dem gelösten Polymer (P) zunächst von dem Lösemittel (LM) abzutrennen und erst anschließend zu einem Medizinprodukt (MED) zu verarbeiten. In diesem Fall umfasst das Verfahren zur Herstellung eines Medizinprodukts (MED) mit einer permanenten Markierung (PM) den Verfahrensschritt Abtrennen des Polymers (P) von dem Lösemittel (LM).

Die Bedingungen unter denen die Verfahrensschritte Lösen des Polymers (P), Verteilen der permanenten Markierung (PM) und Abtrennen des Polymers (P) durchgeführt werden, sind von der Natur des Polymers (P) und dem eingesetzten Lösemittel (LM) abhängig. Diese sind dem Fachmann bekannt und werden nach Bedarf ausgewählt.

Alternativ hierzu ist es möglich das Polymer (P) in einem geeigneten Lösungsmittel (LM) aufquellen zu lassen und so die permanente Markierung (PM) in das Polymer (P) einzuarbeiten. In diesem Fall wird vorzugsweise zunächst die permanente Markierung (PM) in dem Quellmittel (QM) verteilt, insbesondere gelöst oder dispergiert und anschließend des Polymer (P) in dem Quellmittel (QM) umfassend die permanente Markierung (PM) aufgequollen. Anschließend wird das Polymer (P) von dem Quellmittel (QM) abgetrennt.
Die Bedingungen unter denen die permanente Markierung (PM) in dem Quellmittel (QM) verteilt, insbesondere gelöst oder dispergiert werden kann, die Bedingungen unter denen das Polymer (P) in dem Quellmittel (QM)umfassend die permanente Markierung (PM) aufgequollen werden kann und die Bedingungen unter denen das Polymer (P) von dem Quellmittel (QM)abgetrennt werden kann sind von der Natur des Polymers (P) und dem eingesetzten Quellmittel (QM) abhängig. Diese sind dem Fachmann bekannt und werden nach Bedarf ausgewählt.

Eine Schwierigkeit die sich durch den Einsatz der permanenten Markierung (PM) enthaltend mindestens eine Nukleinsäure (NUK) mit einer bekannten Teilsequenz (TSEQ) ergeben kann, ist die geringe Löslichkeit die Nukleinsäuren in den im Zusammenhang mit Polymeren und Polymerisationsprozessen typischerweise eingesetzten organischen Lösemitteln.

In gelöster Form lassen sich die Nukleinsäuren jedoch deutlich homogener in das Polymer einarbeiten.

Eine Möglichkeit in diesem Zusammenhang besteht in der Modifizierung der Nukleinsäuren, um die Löslichkeit in organischen Lösemitteln zu erhöhen. Es ist beispielsweise möglich die Nukleinsäuren mit organischen Kationen zu komplexieren. Ein entsprechendes Verfahren ist in der WO 2013/053483 beschrieben. Geeignete Nukleinsäuren und organische Kationen, sowie ein Verfahren zur Modifizierung sind beispielsweise auf Seite 9, Absatz 2 bis Seite 12, Absatz 1 der WO 2013/053483 beschrieben.

Eine weitere Schwierigkeit ergibt sich durch die Tendenz der Nukleinsäuren sich zu zersetzen und/oder abzubauen. Eine Zersetzung bzw. der Abbau der Nukleinsäuren ist beispielsweise durch die Einwirkung von Säuren oder Basen, die Einwirkung von Enzymen und die Einwirkung erhöhter Temperaturen möglich.

Insbesondere das Polymerisieren des mindestens einem Monomer (M) wird in der Regel bei einer erhöhten Temperatur durchgeführt.

In einer Ausführungsform wird das Polymerisieren des mindestens einem Monomer (M) in einem bestimmten Temperaturbereich durchgeführt, bevorzugt in einem Temperaturbereich von 0 bis 250 °C, weiter bevorzugt in einem Temperaturbereich von 20 bis 200 °C, noch weiter bevorzugt in einem Temperaturbereich von 25 bis 150 °C, noch weiter bevorzugt in einem Temperaturbereich von 30 bis 120 °C, noch weiter bevorzugt in einem Temperaturbereich von 35 bis 110 °C.

Da die in der permanenten Markierung (PM) eingesetzte Nukleinsäure (NUK) auch während anderer Verfahrensschritten dazu neigt sich zu zersetzen und/oder abgebaut wird, ist es bevorzugt, dass keiner der Verfahrensschritte bei dem hier beschriebenen Verfahren zur Authentifizierung eines Medizinprodukts (MED), beziehungsweise bei dem hier beschriebenen Verfahren zur Herstellung eines Medizinprodukts (MED) bei einer Temperatur oberhalb von 250 °C, vorzugsweise oberhalb von 200 °C, weiter bevorzugt oberhalb von 150 °C, noch weiter bevorzugt oberhalb von 120 °C, insbesondere oberhalb von 110 °C, durchgeführt wird.

Gegebenenfalls können weitere Verfahrensschritte vorgesehen sein, wie beispielsweise ein Verfahrensschritt der zu einer Vernetzung des Polymers (P) führt.

Der Begriff "Vernetzung" bezeichnet eine Reaktion, bei der eine Vielzahl einzelner Makromoleküle zu einem dreidimensionalen Netzwerk verknüpft wird. Dies umfasst sowohl eine intramolekulare Vernetzung als auch eine intermolekulare Vernetzung. Die Verknüpfung kann entweder direkt während der Polymerisation und/oder durch eine der Polymerisation nachgelagerte Reaktion erreicht werden. Verfahren zur Herstellung von vernetzten Polymeren sind dem Fachmann bekannt und werden bei Bedarf durchgeführt. Insofern die "Vernetzung" an bereits gebildeten Makromolekülen erfolgt, wird auch von einer "Quervernetzung" gesprochen, was von dem Begriff "Vernetzung" ebenfalls erfasst wird. Im Polymerbereich wird in diesem Zusammenhang auch von der "Härtung" oder dem "Aushärten" gesprochen.

Neben einer Veränderung der mechanischen und physikalischen Eigenschaften der Polymere selbst, verringert eine Vernetzung die Tendenz der permanenten Markierung (PM) unkontrolliert aus dem Polymer auszutreten. Dies kann insbesondere dann von Bedeutung sein, wenn das zur Herstellung des Medizinprodukts (MED) verwendete Polymer (P) umfassend die permanente Markierung (PM) bei der Anwendung am Menschen in direktem Kontakt zum Menschen steht.

Wie oben bereits erläutert ist das Verfahren zur Herstellung des Medizinprodukts (MED) mit einer permanenten Markierung (PM) von dem Medizinprodukt selbst abhängig. Ist ein zusätzlicher Verfahrensschritt zur Vernetzung des Polymers (P) erwünscht, können die beschriebenen Verfahren entsprechend modifiziert werden.

In einer Ausführungsform kann das Verfahren die folgenden Schritte aufweisen:
(i) Bereitstellen von mindestens einem Monomer (M);
(ii) Bereitstellen einer permanenten Markierung (PM) enthaltend mindestens eine Nukleinsäure (NUK) mit einer bekannten Teilsequenz (TSEQ);
(iii) Verteilen der permanenten Markierung (PM) in dem mindestens einem Monomer (M);
(iv) Polymerisieren des mindestens einen Monomers (M), so dass ein Polymer (P) umfassend die permanente Markierung (PM) erhalten wird;
(v) Vernetzen des Polymers (P);
(vi) Verarbeiten des Polymers (P) zu einem Medizinprodukt (MED),
wobei die Vernetzung des Polymers (P) während und/oder nach dem polymerisieren des mindestens einen Monomers (M) erfolgen kann.

In einer weiteren Ausführungsform kann das Verfahren die folgenden Schritte aufweisen:
(i) Bereitstellen eines Polymers (P);
(ii) Bereitstellen einer permanenten Markierung (PM);
(iii) Verteilen der permanenten Markierung (PM) in dem Polymer (P);
(iv) Vernetzten des Polymers (P);
(v) Verarbeiten des Polymers (P) zu einem Medizinprodukt (MED).

In dieser Ausführungsform ist es möglich das Polymer zunächst zu lösen, um die Verteilung der permanenten Markierung (PM) und die anschließend durchgeführte Vernetzung zu erleichtern.

In diesem Fall kann das Verfahren die folgenden Schritte aufweisen:
(i) Bereitstellen eines Polymers (P);
(ii) Bereitstellen eines Lösemittels (LM);
(iii) Lösen des Polymers (P) in dem Lösemittel (LM);
(iv) Bereitstellen einer permanenten Markierung (PM);
(v) Verteilen der permanenten Markierung (PM) in dem gelösten Polymer (P);
(vii) Vernetzten des Polymers (P);
(ix) Verarbeiten des Polymers (P) zu einem Medizinprodukt (MED).

Es kann vorgesehen sei das Polymer (P) nach dem Verteilen der permanenten Markierung (PM) in dem gelösten Polymer (P) und/oder dem Vernetzen das Polymer (P) zunächst von dem Lösemittel (LM) abzutrennen und erst anschließend zu Vernetzen und/oder zu einem Medizinprodukt (MED) zu verarbeiten. In diesem Fall umfasst das Verfahren zur Herstellung eines Medizinprodukts (MED) mit einer permanenten Markierung (PM) den Verfahrensschritt Abtrennen des Polymers (P) von dem Lösemittel (LM).

Die Bedingungen unter denen die Verfahrensschritte Lösen des Polymers (P), Verteilen der permanenten Markierung (PM) und Abtrennen des Polymers (P) durchgeführt werden, sind von der Natur des Polymers (P) und dem eingesetzten Lösemittel (LM) abhängig. Diese sind dem Fachmann bekannt und werden nach Bedarf ausgewählt.

Das Verfahren zur Herstellung des Medizinprodukts (MED) kann also einen Vernetzungsschritt umfassen, welcher während und/oder im Anschluss an die Polymerisation durchgeführt wird.

In einer Ausführungsform ist das in dem Medizinprodukt (MED) verarbeitete Polymer (P) vernetzt. In diesem Fall kann das Verfahren zur Herstellung des Medizinprodukts (MED) einen zusätzlichen Verfahrensschritt umfassen, in dem das Polymer (P) vernetzt wird. Dieser kann während der Polymerisation des mindestens einen Monomers (M) durchgeführt werden und/oder im Anschluss an die Polymerisation an dem Polymer (P). Alternativ hierzu kann in dem Verfahren zur Herstellung eines Medizinprodukts (MED) ein Polymer eingesetzt werden welches bereits vernetzt ist.

In einer Ausführungsform wird die Vernetzung in einem bestimmten Temperaturbereich durchgeführt, bevorzugt in einem Temperaturbereich von 0 bis 250 °C, weiter bevorzugt in einem Temperaturbereich von 20 bis 200 °C, noch weiter bevorzugt in einem Temperaturbereich von 25 bis 150 °C, noch weiter bevorzugt in einem Temperaturbereich von 30 bis 120 °C, noch weiter bevorzugt in einem Temperaturbereich von 35 bis 110 °C.

Der Begriff "Polymer (P)" bezeichnet eine Substanz die aus solchen Molekülen aufgebaut ist, in denen eine Art oder mehrere Arten von Atomen oder Atom-Gruppierungen wiederholt aneinandergereiht sind. In anderen Worten sind Polymere chemische Stoffe aus Makromolekülen, welche aus einer oder mehreren Struktureinheiten sich wiederholenden Struktureinheiten (Monomeren) aufgebaut ist. Es werden sowohl synthetische als auch natürliche Polymere umfasst, insbesondere synthetische oder natürliche Homopolymere, welche aus einer Monomer-Einheit aufgebaut sind und synthetische oder natürliche Copolymere, welche aus zwei oder mehr unterschiedlichen Monomer-Einheiten aufgebaut sind.

Das Polymer (P) das für die Herstellung des Medizinprodukts (MED) eingesetzt wird ist nicht kritisch, solange es für die vorgesehene Anwendung des Medizinprodukts (MED) geeignet ist und unter Bedingungen hergestellt werden kann, welche nicht dazu führen das die in der permanenten Markierung (PM) enthaltenen Nukleinsäure (NUK) mit einer bekannten Teilsequenz (TSEQ) wesentlich verändert wird, insbesondere fragmentiert.

Auch wenn es sich bei einer Nukleinsäure strenggenommen auch um ein Polymer handelt bezeichnet der Begriff "Polymer" im Rahmen dieser Erfindung keine Nukleinsäure. In anderen Worten, sind die Begriffe "Polymer" und " Nukleinsäure" im Rahmen dieser Erfindung nicht gleichzusetzten. Das Polymer (P) unterscheidet sich von der Nukleinsäure (NUK) strukturell. Insbesondere umfasst das Polymer (P) keine Nukleoside und Nukleotide.

Das Polymer (P) kann beispielsweise ausgewählt sein aus der Gruppe bestehend aus Polyolefine, Polyhalogenolefine, Styrolpolymere, Vinylpolymere, Fluorpolymere, Polyacryl- und Methacrylpolymere, Polyoxymethylen, Polyamide, aromatische Polyester, aromatische Polysulfide, aromatische Polyether, aliphatische Polyester, Poly(aryl)etherketone, aromatische Polyimide, Polyurethane, natürlich vorkommende Polymere und Derivate, Kautschuke, aliphatische Polyketone und deren Mischungen.

Entsprechende Polymere sind beispielsweise in Saechtling Kunststoff Taschenbuch, 30. Ausgabe, 2007 Carl Hanser Verlag München, auf Seiten 445-666 beschrieben. Das Polymer (P) kann aus einer einzelnen Art von Monomer (M) aufgebaut sein, indem das Monomer (M) in einer Polymerisationsreaktion zu Polymer (P) umgesetzt wird. Dabei wird ein Homopolymer erhalten. Es ist allerdings auch möglich, dass das Polymer (P) aus zwei oder mehr verschiedenen Monomere (M) aufgebaut wird, indem zwei oder mehr verschiedenen Monomere (M) in einer Polymerisationsreaktion zu Polymer (P) umgesetzt werden. In diesem Fall wird ein Copolymer erhalten. Es werden deshalb neben den entsprechenden Homopolymeren der vorgenannten Polymerklassen auch entsprechende Copolymere erfasst.

Die Natur des eingesetzten Polymers ist von den gewünschten Eigenschaften des Medizinprodukts (MED) abhängig und wird von dem Fachmann entsprechend ausgesucht.

In einer bevorzugten Ausführungsform ist das Polymer (P) ein Kautschuk, insbesondere ein Silikon-Kautschuk und/oder Silikon-Kautschuk Derivate.

Der Silikon-Kautschuk kann ausgewählt werden aus der Gruppe bestehend aus Polydimethylsiloxan-Kautschuk, Polydiphenylsiloxan-Kautschuk, Methly-Phenyl-Siloxan-Kautschuk, Methyl-Vinyl-Siloxan-Kautschuk, Methyl-Phenyl-Vinyl-Siloxan-Kautschuk, Methyl-Fluor-Siloxan-Kautschuk, entsprechende Derivate und deren Mischungen.

Insbesondere kann der Silikon-Kautschuk ausgewählt werden aus der Gruppe bestehend aus Polydimethylsiloxan, Polydiphenylsiloxan, Polymethylphenylsiloxan, Polydimethylsiloxan-co-methylhydrosiloxan, Polydimethylsiloxan mit endständigen Vinylgruppen, Polydiphenylsiloxan mit endständigen Vinylgruppen, Polymethylphenylsiloxan mit endständigen Vinylgruppen, Polydimethylsiloxan-co-methylhydrosiloxan mit endständigen Vinylgruppen, Polydimethylsiloxan mit endständigen Trimethylsilygruppen, Polydiphenylsiloxan mit endständigen Trimethylsilygruppen, Polymethylphenylsiloxan mit endständigen Trimethylsilygruppen, Polydimethylsiloxan-co-methylhydrosiloxan mit endständigen Trimethylsilygruppen und deren Mischungen.

In einer bevorzugten Ausführungsform ist das Polymer (P) vernetzt und aufgebaut aus Polydimethylsiloxan-co-methylhydrosiloxan mit endständigen Trimethylsilygruppen und Polydimethylsiloxan mit endständigen Vinylgruppen. Die Vernetzung des Polymers (P) erfolgt über die endständigen Vinylgruppen.

Die für die Herstellung der vorgenannten Polymere (P) benötigten Monomere (M) sind dem Fachmann bekannt und werden nach Bedarf ausgewählt.

Die Eigenschaften des Medizinprodukts (MED), beziehungsweise die Eigenschaften des Polymers (P), sollen durch die permanente Markierung (PM) möglichst nicht verschlechtert werden. Um die Kompatibilität zwischen der permanenten Markierung (PM) und dem Polymer (P) zu erhöhen, kann es vorgesehen sein, dass die permanente Markierung (PM) zunächst mit einem Polymer (BP) beschichtet und erst anschließend in dem Monomer (M) verteilt wird. Eine Beschichtung der permanenten Markierung (PM) kann auch vorgesehen sein, falls die permanente Markierung (PM) erst im Anschluss an die Polymerisation in das Polymer eingearbeitet wird.

Das für die Beschichtung vorgesehene Polymer (BP) kann dem Polymer (P) entsprechen oder ein von dem Polymer (P) unterschiedliches Polymer sein, dass sich gut verteilen lässt ohne die Eigenschaften des Polymers (P) negativ zu beeinflussen.

Typischerweise umfasst ein so modifiziertes Verfahren einen Schritt bei dem die permanente Markierung (PM) mit dem Polymer (BP) beschichtet wird. Erst im Anschluss an diesen Beschichtungsschritt wird die permanente Markierung (PM) in dem Monomer (M) oder Polymer (P) verteilt.

In einer Ausführungsform umfasst das beschriebene Verfahren einen Schritt bei dem bei dem die permanente Markierung (PM) zunächst mit dem Polymer (BP) beschichtet wird und erst im Anschluss an die Beschichtung mit dem Polymer (BP) in dem Polymer (P) verteilt wird.

In einer anderen Ausführungsform umfasst das beschriebene Verfahren einen Schritt bei dem die permanente Markierung (PM) zunächst mit dem Polymer (BP) beschichtet wird und erst im Anschluss an die Beschichtung mit dem Polymer (BP) in dem Monomer (M) verteilt wird.

Die fälschungssichere Kennzeichnung des Medizinprodukts (MED) erfolgt über eine permanente Markierung (PM), welche mindestens eine Nukleinsäure (NUK) mit einer bekannten Teilsequenz (TSEQ) enthält.

Das Konzept der fälschungssicheren Kennzeichnung von Wertgegenständen durch die oberflächliche Auftragung von Nukleinsäuren ist bereits bekannt.

Im Gegensatz hierzu basiert das beschriebene Verfahren nicht auf einer nachträglich oberflächlich aufgetragenen Markierung, sondern ist auf ein Konzept gerichtet, bei dem die Markierung in einer Polymermatrix vorliegt, welche zur Herstellung des Medizinprodukts eingesetzt wird. In anderen Worten wird die Markierung nicht nachträglich aufgetragen, sondern bereits während dem Herstellungsprozess des Medizinprodukts zugesetzt und in das Medizinprodukt selbst eingearbeitet.

Die fälschungssichere Kennzeichnung des Medizinprodukts (MED) erfolgt über eine permanente Markierung (PM), welche mindestens eine Nukleinsäure (NUK) mit einer bekannten Teilsequenz (TSEQ) enthält. Dazu wird zunächst ein Polymer (P) das zur Herstellung des Medizinprodukts (MED) eingesetzt wird mit der permanenten Markierung (PM), welche mindestens eine Nukleinsäure (NUK) mit einer bekannten Teilsequenz (TSEQ) enthält gekennzeichnet. Die Authentifizierung des Medizinprodukts (MED) erfolgt durch Isolierung und Nachweis der mindestens eine Nukleinsäure (NUK) mit einer bekannten Teilsequenz (TSEQ), welche in der permanenten Markierung (P) enthalten ist.

Die Markierung wird in einem Medizinprodukt eingesetzt. Es ist aufgrund einer potentiellen gesundheitlichen Gefährdung nicht erwünscht, dass die Markierung unkontrolliert aus dem Medizinprodukt (MED) austritt, insbesondere über einen längeren Zeitraum ausblutet.

Wie oben bereits erläutert, ist die permanente Markierung (PM) vorzugsweise dauerhaft mit dem Medizinprodukt verbunden und kann ohne Hilfsmittel nicht in wesentlichen Mengen aus dem Medizinprodukt (MED) isoliert werden. In anderen Worten kann die permanente Markierung (PM) nicht aus dem Medizinprodukt (MED) isoliert werden, ohne dieses über Hilfsmittel strukturell zu verändern, beispielsweise über das Lösen in einem geeigneten Lösemittel, den Abbau des Polymers mit geeigneten Reagenzien oder der mechanischen Entnahme durch eine gezielte äußerliche Einwirkung.

In einer Ausführungsform wird die permanente Markierung (PM) durch den vollständigen Kontakt des Medizinprodukts (MED) mit TE-Puffer (10 mM Tris-HCl, 1 mM EDTA, pH 8.0) nicht in einer Menge über 0,01 Gewichts %/h, bevorzugt in einer Menge über 0,001 Gewichts %/h, weiter bevorzugt in einer Menge über 0,001 Gewichts %/h, noch weiter bevorzugt in einer Menge über 0,00001 Gewichts %/h aus dem Medizinprodukt (MED) freigesetzt, bezogen auf das Gesamtgewicht der permanenten Markierung (PM).

Die Auswahl der Nukleinsäure ist unkritisch, solange sie eine charakteristische Teilsequenz enthält die sich als Markierungsträger eignet. Die Nukleinsäure kann aus einer natürlichen Quelle gewonnen werden oder synthetischen Ursprungs sein. Sowohl einzelsträngige als auch mehrsträngige Nukleinsäuren können in dem beschrieben Verfahren eingesetzt werden. Die Anzahl der Nukleotide ist unkritisch, solange eine Teilsequenz auftritt, die sich als Markierungsträger eignet und entsprechend nachgewiesen werden kann.

Die Nukleinsäure (NUK) mit einer bekannten Teilsequenz (TSEQ) ist eine natürlich vorkommende Nukleinsäure, eine synthetisch hergestellte Nukleinsäure, oder ein Gemisch aus einer natürlich vorkommenden Nukleinsäure und einer synthetisch hergestellten Nukleinsäure. Bei der Nukleinsäure (NUK) mit einer bekannten Teilsequenz (TSEQ) kann es sich um Desoxyribunukleinsäure (DNA) oder Ribonukleinsäure (RNA) handeln, oder auch um Derivate, die ganz oder teilweise seltene Basen oder modifizierte Basen enthalten. In einer bevorzugten Ausführungsform ist die Nukleinsäure (NUK) mit einer bekannten Teilsequenz (TSEQ) eine Desoxyribunukleinsäure (DNA).

In einer bevorzugten Ausführungsform ist die Nukleinsäure (NUK) mit einer bekannten Teilsequenz (TSEQ) eine synthetisch hergestellte Nukleinsäure.

Die Nukleinsäure (NUK) mit einer bekannten Teilsequenz (TSEQ) kann eine einzelsträngige Nukleinsäure, eine doppelsträngige Nukleinsäure, oder ein Gemisch aus einer einsträngigen Nukleinsäure und einer doppelsträngigen Nukleinsäure sein.

In einer bevorzugten Ausführungsform ist die Nukleinsäure (NUK) mit einer bekannten Teilsequenz (TSEQ) eine doppelsträngige Nukleinsäure.

In einer Ausführungsform ist die Nukleinsäure (NUK) mit einer bekannten Teilsequenz (TSEQ) eine doppelsträngige, synthetisch hergestellte Nukleinsäure mit einer Anzahl an Nukleotiden in einem Bereich von 10 bis 10 000, vorzugsweise in einem Bereich von 10 bis 1000, noch weiter bevorzugt in einem Bereich von 15 bis 250.

Um die Fälschungssicherheit zu erhöhen ist es möglich neben der Nukleinsäure (NUK) mit einer bekannten Teilsequenz (TSEQ) weitere Nukleinsäuren vorzusehen, welche der Nukleinsäure (NUK) mit einer bekannten Teilsequenz (TSEQ) ähnlich sind, aber die für die Authentifizierung erforderliche Teilsequenz (TSEQ) nicht aufweisen und deshalb für jemanden der die Teilsequenz (TSEQ) nicht kennt nur schwer von der Nukleinsäure (NUK) mit einer bekannten Teilsequenz (TSEQ) unterscheidbar sind.

In einer Ausführungsform umfasst die permanente Markierung (PM) neben der Nukleinsäure (NUK) mit einer bekannten Teilsequenz (TSEQ) mindestens eine weitere Nukleinsäure, welche die Teilsequenz (TSEQ) nicht aufweist.

Diese mindestens eine weitere Nukleinsäure kann in der permanenten Markierung (PM) enthalten sein in einem Verhältnis Nukleinsäure zu Nukleinsäure (NUK) mit einer bekannten Teilsequenz (TSEQ) in Bereich von 1/100 bis 100/1, vorzugsweise 1/50 bis 50/1, weiter bevorzugt 1/10 bis 10/1.

In einer Ausführungsform ist die mindestens eine weitere Nukleinsäure im Vergleich zu der Nukleinsäure (NUK) mit einer bekannten Teilsequenz (TSEQ) in der permanenten Markierung in mindestens in gleiche Menge enthalten, um so die zur Authentifizierung vorgesehene Teilsequenz (TSEQ) effektiv zu "verstecken". In diesem Fall kann das Verhältnis Nukleinsäure zu Nukleinsäure (NUK) mit einer bekannten Teilsequenz (TSEQ) in Bereich von 100/1 bis 1/1, vorzugsweise 50/1 bis 1/1, weiter bevorzugt 30/1 bis 2/1, noch weiter bevorzugt 10/1 bis 3/1.

Die Authentifizierung des Medizinprodukts (MED) mit der permanenten Markierung (PM) erfolgt über die bekannte Teilsequenz (TSEQ) der Nukleinsäure (NUK), welche in der permanenten Markierung (PM) vorgesehen ist. Dazu ist es erforderlich die permanente Markierung (PM) aus dem Medizinprodukt (MED) zu isolieren.

Wie bereits erläutert kann die permanente Markierung (PM) vorzugsweise nicht ohne Hilfsmittel aus dem Medizinprodukt (MED) isoliert werden.

Um die permanente Markierung (PM) aus dem Medizinprodukt (MED) zu isoliert, ist es beispielsweise möglich das Medizinprodukt (MED) zunächst in einem geeigneten Lösemittel aufzulösen und anschließend die so freigesetzte permanente Markierung (PM) zu isolieren. Außerdem ist es möglich die permanente Markierung (PM) mit einem geeigneten Werkzeug durch äußerliche Einwirkung aus dem Medizinprodukt (MED) zu entfernen.

Gemäß der vorliegenden Erfindung ist unter Isolierung der permanenten Markierung (PM) zu verstehen, dass die permanente Markierung (PM) für den Nachweis der mindestens einen bekannten Teilsequenz (TSEQ) zugänglich gemacht wird.

Es kann erforderlich sein das die permanente Markierung (PM) zunächst in dem Medizinprodukt (MED) lokalisiert werden muss. Die Lokalisierung der permanenten Markierung (PM) in dem Medizinprodukt (MED) kann durch das Vorsehen eines zusätzlichen Merkmals erleichtert werden. Es ist beispielsweise möglich die permanente Markierung (PM) so zu gestalten, dass sie optisch lokalisiert werden kann.

Die Nukleinsäure (NUK) mit einer bekannten Teilsequenz (TSEQ) kann zunächst mit einem Farbmittel behandelt und erst anschließend in dem Polymer (P) verteilt werden. Geeignete Farbmittel zur schonenden Behandlung von Nukleinsäuren sind dem Fachmann bekannt und werden nach Bedarf ausgesucht.

Die Nukleinsäure (NUK) mit einer bekannten Teilsequenz (TSEQ) kann mit einem Farbmittel behandelt werden. Geeignete Farbmittel sind dem Fachmann bekannt und werden nach Bedarf ausgesucht. Beispielsweise kann *N'*,*N'*,2-Trimethylphenothiazin-3,7-diaminchlorid als Farbmittel verwendet werden.
Für die Authentifizierung des Medizinprodukts (MED) wird die permanente Markierung (PM) zunächst mit geeigneten Hilfsmitteln isoliert. Das Medizinprodukt (MED) ist authentisch wenn die mindestens eine bekannte Teilsequenz (TSEQ) in der mindestens einen Nukleinsäure (NUK), welche in der permanenten Markierung (PM) enthalten ist, nachgewiesen werden kann.

Dem Fachmann sind geeignete Methoden zum Nachweis der Teilsequenz einer Nukleinsäure bekannt und werden nach Bedarf ausgewählt. Dabei kann eine solche sequenzspezifische Nachweismethode eine Vervielfältigung (Amplifikation) der mindestens einen bekannten Teilsequenz (TSEQ) in der mindestens einen Nukleinsäure (NUK) umfassen. Dem Fachmann sind geeignete Methoden zur Amplifikation von Nukleinsäuren bekannt und werden nach Bedarf ausgewählt. Geeignete Methoden zur Amplifikation sind beispielsweise die Polymerase-KettenReaktion und die isothermale Amplifikation.

Bei einer Amplifikation von Nukleinsäuren wird mittels spezifischer Primer eine bekannten Teilsequenz (TSEQ) einer Nukleinsäure (NUK) vervielfältigt. Unter einem spezifischen Primer wird ein durch Polymerase verlängerbares Oligonukleotid verstanden, welches unter geeigneten Hybridisierungsbedingungen spezifisch mit einer Nukleinsäure hybridisiert. Dem Fachmann sind geeignete Methoden zur Auswahl eines spezifischen Primers bekannt und werden nach Bedarf ausgewählt. Die so amplifizierte Teilsequenz der Nukleinsäure weist nicht nur eine spezifische Abfolge von Nukleotiden (Sequenz) sondern auch eine spezifische Anzahl an Nukleotiden (Länge) auf.

Um die bekannte Teilsequenz sequenzspezifisch nachzuweisen, kann also in einem ersten Schritt die mindestens einen bekannten Teilsequenz (TSEQ) in der mindestens einen Nukleinsäure (NUK) mittels spezifischer Primer amplifiziert werden und in einem weiteren Schritt kann die Länge der amplifizierten Nukleinsäure bestimmen werden. Dem Fachmann sind geeignete Methoden zur Bestimmung der Länge einer Nukleinsäure bekannt und werden nach Bedarf ausgewählt. Eine geeignete Methode zur Bestimmung der Länge einer Nukleinsäure ist beispielsweise die Gelelektrophorese.

Abhängig von der Menge an isolierter permanenter Markierung (PM) und der Methoden zum Nachweis der Teilsequenz einer Nukleinsäure, kann die mindestens eine bekannten Teilsequenz (TSEQ) aber auch direkt, also ohne Amplifikation der bekannten Teilsequenz (TSEQ), sequenzspezifisch nachgewiesen werden. Dem Fachmann sind geeignete Methoden zum direkten Nachweis der Teilsequenz einer Nukleinsäure bekannt und werden nach Bedarf ausgewählt.

Geeignete Methoden zum direkten Nachweis sind beispielsweise die Sequenzierung, also die Bestimmung der Nukleotid-Abfolge. Die so ermittelte Sequenz kann dann mit der bekannten Teilsequenz (TSEQ) abgeglichen werden. Dem Fachmann sind geeignete Methoden zur Ermittlung der Sequenz einer Nukleinsäure bekannt und werden nach Bedarf ausgewählt.

Ein direkter Nachweis kann auch durch Hybridisierung mit einer Nachweisnukleinsäure, die zumindest partiell zu der bekannten Teilsequenz (TSEQ) der Nukleinsäure (NUK) komplementär ist, erfolgen.

Abhängig von der Methode zum Nachweis der Teilsequenz einer Nukleinsäure kann die Nachweisnukleinsäure unmarkiert oder markiert sein. Dem Fachmann sind geeignete Nachweisnukleinsäuren bekannt und werden nach Bedarf ausgewählt. Beispielsweise kann die Nachweisnukleinsäure ein "Molecular Beacon" sein, welches mit einem Fluorszenzfarbstoff und einem Quencher markiert ist. Bei einer Hybridisierung eines "Molecular Beacon" mit einer komplementären Sequenz ändern sich die Fluoreszenzeigenschaften. Diese Änderung der Fluoreszenzeigenschaften kann als sequenzspezifischer Nachweis der bekannten Teilsequenz einer Nukleinsäure genutzt werden.
Dem Fachmann ist bekannt, dass sequenzspezifische Nachweismethoden oder auch einzelne Schritte der sequenzspezifischen Nachweismethoden kombiniert werden können. So kann beispielsweise die mindestens einen bekannten Teilsequenz (TSEQ) in der mindestens einen Nukleinsäure (NUK) in einem ersten Schritt mittels sequenzspezifischer Primer amplifiziert werden und in einem weiteren Schritt mit einer der oben beschriebenen direkten Nachweismethoden kombiniert werden.

Der Nachweis der bekannten Teilsequenz kann qualitativ und/oder quantitativ erfolgen. Erfolgt ein quantitativer Nachweis, so erlaubt das erfindungsgemäße Verfahren neben einer qualitativen Authentifizierung des Medizinprodukts (MED) auch eine quantitative Authentifizierung, also die Feststellung, ob das Polymer (P), welches die permanente Markierung (PM) umfasst, mit einem strukturell ähnlichen oder identischen Polymer vermengt beziehungsweise verdünnt wurde. In anderen Worten ist es mit dem erfindungsgemäßen Verfahren möglich eine quantitative Authentifizierung des Medizinprodukts (MED) durchzuführen und somit über das mit der permanenten Markierung (PM) gekennzeichnete Polymer (P) festzustellen, ob es mit einem strukturell ähnlichen oder identischen Polymer "gestreckt" wurde.

Dem Fachmann sind geeignete quantitative sequenzspezifische Nachweismethoden bekannt und werden nach Bedarf ausgewählt. Beispielsweise kann der Nachweis eine quantitative Polymerase-Kettenreaktion umfassen. Ebenso kann ein quantitativer Nachweis der bekannten Teilsequenz beispielsweise die Verwendung eines Molecular Beacons umfassen, da die Änderung der Fluoreszenzeigenschaften, die durch die Hybridisierung hervorgerufenen werden, nicht nur einen qualitativen, sondern auch einen quantitativen Nachweis erlauben.

Für eine quantitative Authentifizierung des Medizinprodukts (MED) ist es zunächst erforderlich die permanente Markierung (PM) unter Berücksichtigung eines gewissen Toleranzbereichs möglichst homogen in dem Polymer (P) zu verteilen. Zu einem späteren Zeitpunkt wird dann das Medizinprodukt (MED) über die in der permanenten Markierung enthalten Nukleinsäure (NUK) mit bekannter Teilsequenz (TSEQ) qualitativ und quantitativ authentifiziert. Dabei wird die Konzentration der permanenten Markierung (PM) in dem Polymer (P) bestimmt und mit der Konzentration der permanenten Markierung (PM) in dem zur Herstellung des Medizinprodukts (MED) eingesetzten Polymer (P) verglichen. Liegt die Abweichung innerhalb des Toleranzbereichs ist davon auszugehen, dass das Polymer (P) nicht mit einem strukturell ähnlichen oder identischen Polymer vermengt beziehungsweise verdünnt wurde.

In einer Ausführungsform umfasst das Verfahren zur Authentifizierung des Medizinprodukts (MED) zusätzlich die folgenden Schritte:
(i) Bestimmen der Konzentration (EK) der permanenten Markierung (PM) in dem Polymer (P);
(ii) Vergleichen der Konzentration (EK) der permanenten Markierung mit der Konzentration (AK) der permanenten Markierung (PM) des zur Herstellung des Medizinprodukts (MED) verwendeten Polymers (P).

In einer Ausführungsform wird zur Bestimmung der Konzentrationen (EK) und (AK) ein definiertes Volumen (V) des Polymers (P) herangezogen, beispielsweise ein Volumen (V) von 0,01 bis 30 cm³, vorzugsweise ein Volumen von 0,05 bis 10 cm³, weiter bevorzugt ein Volumen von 0,1 bis 3 cm³.

In einer Ausführungsform umfasst der Toleranzbereich bei der Bestimmung der Konzentrationen (EK) und (AK) eine Abweichung von bis zu 5 Gew.-%, vorzugsweise eine Abweichung von bis zu 2 Gew.-%, weiter bevorzugt eine Abweichung von bis zu 1 Gew.-%, noch weiter bevorzugt eine Abweichung von bis zu 0,5 Gew.-%, noch weiter bevorzugt eine Abweichung von 0,05 Gew.-%, bezogen auf das Gesamtgewicht der permanenten Markierung (PM) in dem Volumen (V).

Es wird außerdem ein Medizinprodukt (MED) mit der permanenten Markierung (PM), umfassend ein Polymer (P) mit einer permanenten Markierung (PM) beschrieben, wobei die permanente Markierung (PM) mindestens eine Nukleinsäure (NUK) mit einer bekannten Teilsequenz (TSEQ) enthält.

Das im Rahmen des Verfahrens zur Authentifizierung des Medizinprodukts (MED) über das Polymer (P), die Markierung (PM), die Nukleinsäure (NUK) und die Teilsequenz (TSEQ) gesagte, gilt auch für das Medizinprodukt (MED) als solches.

Außerdem wird die Verwendung einer permanenten Markierung (PM), umfassend mindestens eine Nukleinsäure (NUK) mit einer bekannten Teilsequenz (TSEQ) zur Authentifizierung eines Medizinprodukts (MED) beschrieben.

Das im Rahmen des Verfahrens zur Authentifizierung des Medizinprodukts (MED) über die Markierung (PM), die Nukleinsäure (NUK) und die Teilsequenz (TSEQ) gesagte, gilt auch für Verwendung einer permanenten Markierung (PM) zur Authentifizierung eines Medizinprodukts (MED).

### Beispiele

Im Folgenden ist die Authentifizierung eines Silikon-Kautschuks beschrieben. Silikon-Kautschuk kann für die Herstellung eines Medizinprodukts, beispielsweise einer Brustimplantathülle, verwendet werden.

Zur Authentifizierung des Silikon-Kautschuks wurde genomische DNA (gDNA) einer Tomate in ein flüssiges Silikongemisch eingearbeitet. Durch eine platinkatalysierte Vernetzungsreaktion wurde die gDNA permanent in den Silikon-Kautschuk eingebettet. Die Isolierung der permanenten gDNA-Markierung erfolgte mit Hilfe eines Skalpells. Zwei bekannte Teilsequenzen der gDNA wurden durch Polymerase-Kettenreaktion (PCR) und anschließender Gelelektrophorese nachgewiesen.

### Herstellung des Silikon-Kautschuks mit permanenter Markierung

Es wurde ein vernetzbares Zweikomponenten-Flüssigsilikonkautschuksystem (Silbione® LSR 4305 HC von Bluestra Silicones, vernetzt über Pt-Katalysator) verwendet. Das LSR-Kautschuksystem enthielt Polydimethylsiloxan mit Vinylendgruppen und Polydimethylsiloxan-Co-Polymethylhydrosiloxan mit Trimethylsilylendgruppen, welche jeweils als Dispersion in pyrogener hydrophober Kieselsäure eingesetzt wurden.

Für die permanente Markierung wurde gDNA aus Tomaten genutzt. Hierfür wurde aus 2 g Tomatenblättern (Jungpflanzen, Sorte *Tomimaru Muchoo*) gDNA isoliert. Die isolierte gDNA wurde in 500 µl TE-Puffer (10 mM Tris, 1 mM EDTA) gelöst. 10 µl dieser Lösung dienten als Vergleichsprobe für den späteren Nachweis (Probe 1). Nach der Bestimmung der Konzentration wurde diese gefällt und unter Vakuum getrocknet.

In das flüssige Silikongemisch wurde 361 ± 40,6 µg getrocknete gDNA eingearbeitet. Hierfür wurden jeweils 1 g der beiden Komponenten des LSR-Kautschuksystems vermischt und zentrifugiert. Das getrocknete DNA-Pellet wurde vorsichtig zwischen zwei Schichten des flüssigen Silikongemisches platziert, so dass es vollständig von dem flüssigen Silikongemisches umschlossen war. Anschließend wurde das flüssige Silikongemisch unter Vakuum für 8 h bei 105 °C ausgehärtet.

### Bestimmung der Barriereeigenschaften des Silikon-Kautschuks

Das in den Silikon-Kautschuk eingebettete gDNA-Pellet wurde unter dem Mikroskop grobflächig aus dem Material herausgeschnitten. Dabei wurde darauf geachtet, dass die gDNA vollständig von dem Silikon-Kautschuk umschlossen blieb.

Das herausgeschnittene gDNA-Pellet wurde mit etwa 1 ml TE-Puffer vollständig bedeckt. Nach 4 Tagen wurde der TE-Puffer auf herausgelöste DNA-Moleküle untersucht. Hierzu wurden 10 µL des TE-Puffers entnommen (Probe 2) und mittels PCR und anschließender Gelelektrophorese untersucht.

### Isolieren der permanenten Markierung

Die Silikon-Kautschuk-Probe mit dem eingeschlossenen gDNA-Pellet wurde aus dem TE-Puffer entnommen und unter dem Mikroskop, mit Hilfe eines Skalpells, gDNA-Stückchen herausgeschnitten. Diese gDNA-Stückchen (Probe 3) wurden mittels PCR und anschließender Gelelektrophorese untersucht.

### Nachweis einer bekannten Teilsequenz der gDNA

Es wurde eine PCR mit den drei verschiedenen gDNA-Proben (Probe 1, Probe 2 und Probe 3) durchgeführt.

Jede Probe wurde mit zwei verschiedenen Primerpaaren amplifiziert:
- Primerpaar 1: 5'-TACTGGTGGTTTTGAAGCTG-3' (SEQ ID Nr.: 1), 5'-AACTTCCTTCACGATTTCATCATA-3' (SEQ ID Nr.: 2); erwartete Länge der vervielfältigten DNA: 279 Basenpaare (bp);
- Primerpaar 2: 5'-AGACCACGAGAACGATATTTGC-3'(SEQ ID Nr.: 3); 5'-TTCTTGCCTTTTCATATCCAGACA-3' (SEQ ID Nr.: 4); erwartete Länge der vervielfältigten DNA: 92 Basenpaare (bp)

Anschließend wurden die amplifizierten DNA-Abschnitte der PCR-Proben mittels eines 2 %-igen Agarosegels analysiert.

Durch Vergleich mit einem 50 bp Marker zeigte sich, dass die PCR der Vergleichsprobe (Probe 1: gDNA vor Einschluss in den Silikon-Kautschuk) mit dem Primerpaar 1 zu einem vervielfältigten DNA-Abschnitt in einem Größenbereich von 250 bis 300 bp führte. Mit dem Primerpaar 2 wurde ein DNA-Abschnitt in einem Größenbereich von 90 bis 110 bp vervielfältigt. Die Größen der amplifizierten DNA-Abschnitte der Probe 1 stimmten mit jenen der Probe 3 (aus den Silikon-Kautschuk mit dem Skalpell herausgeschnittene DNA) überein. Somit wurde die gDNA während dem Aushärten bei 105 °C für 8h unbeschadet in dem Silikon-Kautschuk eingeschlossen und die bekannte Teilsequenz konnte nach der Isolierung der DNA aus dem Silikon-Kautschuk nachgewiesen werden.

In der Probe 2 (TE-Puffer, der für 4 Tage mit der vollständig umschlossenen DNA-Markierung in Kontakt war) wurden keine DNA-Abschnitte vervielfältigt. Daraus lässt sich ableiten, dass sich die eingeschlossene gDNA nicht aus der Silikonmatrix herauslösen ließ.

### Einfluss der Vernetzungstemperatur

Hierfür wurden die gDNA-Pellets verwendet, die vor der Trocknung im Vakuum mit einer 0.02 %-igen bis 0.12 %-igen Toluidinblau-Lösung (N',N'-2-Trimethylphenothiazin-3,7-diaminchlorid) eingefärbt.

Die gefärbten DNA-Pellets wurden, wie oben beschrieben, in den Silikon-Kautschuk eingebettet. Die Vernetzung wurde bei unterschiedlichen Temperaturen durchgeführt: 105 °C für 8 h, 150 °C für 6 h, 175 °C für 5 h und 200 °C für 4 h.

Bei der Vernetzung bei 105 °C sowie der Vernetzung bei 150 °C h blieb die Färbung der gDNA-Pellets unverändert. Die eingeschlossene gDNA wurde anschließend mit Hilfe eines Skalpells herausgeschnitten. Mittels PCR und anschließender Gelelektrophorese konnten bekannte Teilsequenzen der herausgeschnittenen gDNA wie oben beschrieben nachgewiesen werden.

Bei der Vernetzung bei 175 °C sowie der Vernetzung bei 200 °C änderte sich die Färbung der g-DNA-Pellets. Es wurde bräunlich-schwarz gefärbte g-DNA isoliert, bekannte Teilsequenzen konnten in der herausgeschnittenen gDNA nicht mehr nachgewiesen werden.

### Bestimmung des Anteils an nicht-vernetztem Silikon-Kautschuk

Bei Implantaten kann freies Silikon (nicht-vernetztes Silikon) zu Gesundheitsschäden im Körper des Patienten führen. Daher wurde der nicht-vernetzte Silikonanteil in Abhängigkeit von der Vernetzungstemperatur untersucht.

Die Bestimmung erfolgte mittels einer Soxhlet-Extraktion unter Verwendung von n-Hexan. Das nicht-vernetzte Silikon (freies Silikon) wurde durch das Lösemittel, n-Hexan, aus der Silikonmatrix herausgelöst bzw. extrahiert. Das nicht lösliche, vernetzte Silikon quillte im n-Hexan auf und blieb in der Extraktionshülse.

Der höchste Vernetzungsgrad in der Silikonmatrix wurde bei der geringsten Vulkanisationstemperatur in Verbindung mit der längsten Vulkanisationsdauer (105 °C, 8 h) erreicht. Bei einer Probenmenge von 0,55 g lag der nicht vernetzte Silikonanteil bei 23,8 %. Der niedrigste Vernetzungsgrad in der Silikonmatrix und somit der höchste nicht-vernetzte Silikonanteil, welcher bei 29,1 % lag, ergab sich unter einer Vulkanisationsbedingung von 200 °C für 4 h. Bei 150 °C für 6 h lag der nicht-vernetzte Silikonanteil bei 27,3 %, bei 175°C für 4 h bei 29,0 %.

## Patentansprüche

1. Verfahren zur Authentifizierung eines Medizinprodukts (MED), umfassend die folgenden Schritte:
(i) Herstellen eines Medizinprodukts (MED) mit einer permanenten Markierung (PM), wobei das Medizinprodukt (MED) ein Polymer (P) mit der permanenten Markierung (PM) umfasst und die permanente Markierung (PM) mindestens eine Nukleinsäure (NUK) mit einer bekannten Teilsequenz (TSEQ) enthält;
(ii) Isolieren der permanenten Markierung (PM); und
(iii) Authentifizierung des Medizinprodukts (MED) durch Nachweis der mindestens einen bekannten Teilsequenz (TSEQ).

2. Verfahren gemäß Anspruch 1, wobei der Nachweis
(i) die Amplifikation der mindestens einen bekannten Teilsequenz (TSEQ) der mindestens einen Nukleinsäure (NUK)
und/oder
(ii) die Sequenzierung der mindestens einen bekannten Teilsequenz (TSEQ) der mindestens einen Nukleinsäure (NUK)
und/oder
(iii) die Hybridisierung der mindestens einen bekannten Teilsequenz (TSEQ) der mindestens einen Nukleinsäure (NUK) mit einer Nachweisnukleinsäure
umfasst.

3. Verfahren gemäß einem der vorhergehenden Ansprüche, wobei die Authentifizierung des Medizinprodukts (MED) durch Nachweis der mindestens einen bekannten Teilsequenz (TSEQ) folgende Schritte umfasst:
(i) Bestimmen der Konzentration (EK) der permanenten Markierung (PM) in dem Polymer (P) des zu authentifizierenden Medizinprodukts (MED);
(ii) Vergleichen der Konzentration (EK) der permanenten Markierung mit der Konzentration (AK) der permanenten Markierung (PM) des zur Herstellung des Medizinprodukts (MED) verwendeten Polymers (P).

4. Verfahren zur Herstellung eines Medizinprodukts (MED) mit einer permanenten Markierung (PM) umfassend die folgenden Schritte:
(i) Bereitstellen eines Polymers (P) mit einer permanenten Markierung (PM), wobei die permanente Markierung (PM) mindestens eine Nukleinsäure (NUK) mit mindestens einer bekannten Teilsequenz (TSEQ) enthält; und
(ii) Verarbeiten des Polymers (P) zu einem Medizinprodukt (MED).

5. Verfahren gemäß Anspruch 4, wobei die Herstellung des Medizinprodukts (MED) die folgenden Schritte umfasst:
(a1) Bereitstellen von mindestens einem Monomer (M);
(a2) Bereitstellen einer permanenten Markierung (PM) enthaltend mindestens einen Nukleinsäure (NUK) mit einer bekannten Teilsequenz (TSEQ);
(a3) Verteilen der permanenten Markierung (PM) in dem mindestens einem Monomer (M);
(a4) Polymerisieren des mindestens einen Monomers (M), so dass ein Polymer (P) umfassend die permanente Markierung (PM) erhalten wird; und
(a5) Verarbeiten des Polymers (P) zu einem Medizinprodukt (MED)
oder
(b1) Bereitstellen eines Polymers (P);
(b2) Bereitstellen einer permanenten Markierung (PM);
(b3) Verteilen der permanenten Markierung (PM) in dem Polymer (P); und
(b4) Verarbeiten des Polymers (P) zu einem Medizinprodukt (MED)
oder
(c1) Bereitstellen eines Polymers (P);
(c2) Bereitstellen eines Lösemittels (LM);
(c3) Lösen des Polymers (P) in dem Lösemittel (LM);
(c4) Bereitstellen einer permanenten Markierung (PM);
(c5) Verteilen der permanenten Markierung (PM) in dem gelösten Polymer (P); und
(c6) Verarbeiten des Polymers (P) zu einem Medizinprodukt (MED).

6. Verfahren gemäß einem der vorhergehenden Ansprüche, wobei das in dem Medizinprodukt enthaltene Polymer (P) vernetzt ist.

7. Verfahren gemäß einem der vorhergehenden Ansprüche, wobei die Nukleinsäure (NUK) mit einer bekannten Teilsequenz (TSEQ) eine Desoxyribunukleinsäure (DNA) ist und/oder wobei das Medizinprodukt (MED) ein Medizinprodukt der Klasse III ist.

8. Verfahren gemäß einem der vorhergehenden Ansprüche, wobei die permanente Markierung (PM) neben der Nukleinsäure (NUK) mit einer bekannten Teilsequenz (TSEQ) mindestens eine weitere Nukleinsäure enthält, welche die Teilsequenz (TSEQ) nicht aufweist.

9. Medizinprodukt (MED) mit einer permanenten Markierung (PM), umfassend ein Polymer (P) mit einer permanenten Markierung (PM), wobei die permanente Markierung (PM) mindestens eine Nukleinsäure (NUK) mit einer bekannten Teilsequenz (TSEQ) enthält.

10. Verwendung einer permanenten Markierung (PM), umfassend mindestens eine Nukleinsäure (NUK) mit einer bekannten Teilsequenz (TSEQ) zur Authentifizierung eines Medizinprodukts (MED).
